# EUROPEAN PATENT SPECIFICATION

(11) **EP 3 894 091 B1**
(45) Date of publication and mention of the grant of the patent: **16.08.2023**
(21) Application number: 19823779.4
(22) Date of filing: 12.12.2019
(51) Int. Cl.: B05B 17/06, B06B 1/04, B06B 1/06, B05B 12/00

(54) **INDUCTIVE COUPLING BASED VIBRATION HEADS FOR AN AEROSOL GENERATOR**
AUF INDUKTIVER KOPPLUNG BASIERENDE VIBRATIONSKÖPFE FÜR EINEN AEROSOLGENERATOR
TÊTES DE VIBRATIONS À BASE DE COUPLAGE INDUCTIF POUR UN GÉNÉRATEUR D'AÉROSOL

(30) Priority: 14.12.2018 EP 18212622
(43) Date of publication of application: 20.10.2021
(73) Proprietor: PARI Pharma GmbH, 82319 Starnberg (DE)
(72) Inventor: ACHTZEHNER, Wolfgang, 82239 Alling (DE); REINHART, Markus, 86919 Utting (DE); FINKE, Matthias, 82152 Planegg (DE)
(74) Representative: Hoffmann Eitle
(86) International application number: PCT/EP2019/084869
(87) International publication number: WO 2020/120665

(56) References cited:
- WO-A1-91/16997
- WO-A1-2011/083380
- CN-A- 108 970 891
- KR-U- 20150 003 491
- US-A1- 2012 291 777
- US-A1- 2018 313 556
- US-B2- 9 962 505

## Description

### TECHNICAL FIELD

The present invention relates to a vibration head for an aerosol generator, a controller for an aerosol generator, an aerosol generator, and an electric connecting element, in particular for implementing an inductive coupling based electrical connection for vibration heads of an aerosol generator.

### BACKGROUND

Aerosols for therapeutic purposes are generated and delivered to a desired location within a user's or patient's body with an aerosol delivery device. A fluid or liquid (including a medicament or drug) to be aerosolized or nebulized is supplied to an aerosol generator, the fluid or liquid is aerosolized or nebulized by the aerosol generator and the resultant aerosol is delivered to the user or patient.

The fluid or liquid may be aerosolized or nebulized in the aerosol generator by a vibratable element which is referred to as a vibratable nebulizer, membrane nebulizer, mesh nebulizer, vibration component, or vibration head in the following. Such a vibration head is provided at least with a membrane and an oscillation generator or vibration generating element, such as a piezoelectric element (electromechanical transducer element). The characteristics (mechanical and/or electrical) of the vibration head of the aerosol generator are decisive for the quality of the generated aerosol. At the same time, the vibration head is also generally very sensitive, especially with respect to dimensional specifications. For example, a misalignment of the vibration head may negatively affect the oscillatory or vibration motion of the vibration head during aerosol generation and therefore compromise the quality of the generated aerosol and the dosage accuracy. Also, problems with the electrical connection of the vibration head may lead to problems with the aerosol quality and dosage accuracy.

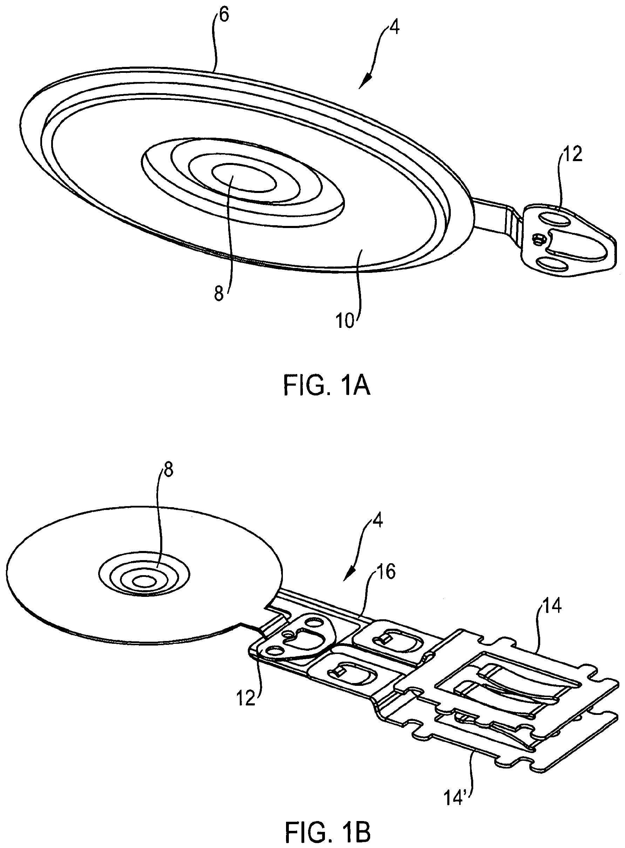

**Figs. 1A** und **1B** respectively show a schematic perspective bottom view and top view of an example of a vibration head of an aerosol generator as disclosed in EP 2 957 349 A1. Here, one side of a vibration head 4 has a support member 6, a vibratable membrane 8 with a plurality of holes (not shown), an annular vibration generating element 10, e.g. a piezoelectric element, and a connection portion 12. The piezoelectric element 10 serves as a vibrator for vibrating the vibratable membrane 8.

In operation, an aerosol (i.e. liquid droplets) is generated on one side of the vibratable membrane 8 from a liquid or fluid that is provided on the other side of the vibratable membrane. In particular, the fluid abutting the membrane 8 is conveyed through the holes or openings (not shown) in the vibrating membrane 8 and is thereby aerosolised into an aerosol cavity or chamber (not shown) of an aerosol delivery device (not shown) arranged below the vibrating membrane 8. The aerosol thus provided in the aerosol cavity or chamber can be inhaled by a user or patient through a mouthpiece, nosepiece, nasal prongs, endotracheal tube, ventilator tube system, and/or face mask (not shown) of the aerosol delivery device.

Further, the vibration head 4 of **Fig. 1B** comprises a pair of electric contacts 14, 14', e.g., plugs for connecting to a controller (not shown). As shown here, the electrical contacts 14, 14' may be punched out from a stainless steel sheet and are subsequently bent, i.e. bent into the shape as shown in **Fig. 1B****.** The electrical contacts are connected here to the connection member 12 and the piezoelectric element 10 through a flexible strip conductor 16, such as a printed board track or a strip line.

The support member 6 and the vibratable membrane 8 as shown in **Fig. 1A** may also be provided as an integrated element (one-pieced) and in such a way a vibration head may also be provided without a support member.

Alternatively, a vibration head may also be provided without a fixed combination of the support member 6 and/or the membrane 8 with the vibration generating element 10, for example in such a case the support member 6 and/or the membrane 8 is replaceably inserted into an aerosol generator by bringing a piezoelectric element into contact with a support member 6 and/or a vibratable membrane of the aerosol generator.

The electrical contacts 14, 14' for the vibration head are not restricted to the example shown in **Fig. 1B** and may, for example, be constructed in a way such that a multiple-wire connection cord for an electric connection with the controller may be connected. In fact, the vibration head may comprise one or more electrical contacts, e.g., pins, plugs, connectors, jacks, clips, cinches or the like, for connection to a control, e.g., an external control. Here, the control may be any type of control, e.g., a control unit, a control element, a control circuit or the like. The control may be capable of electrically operating the vibration head of the aerosol generator. The control may be connectable via the one or more electrical contacts to the vibration head, e.g., to a power supply element of the vibration head.

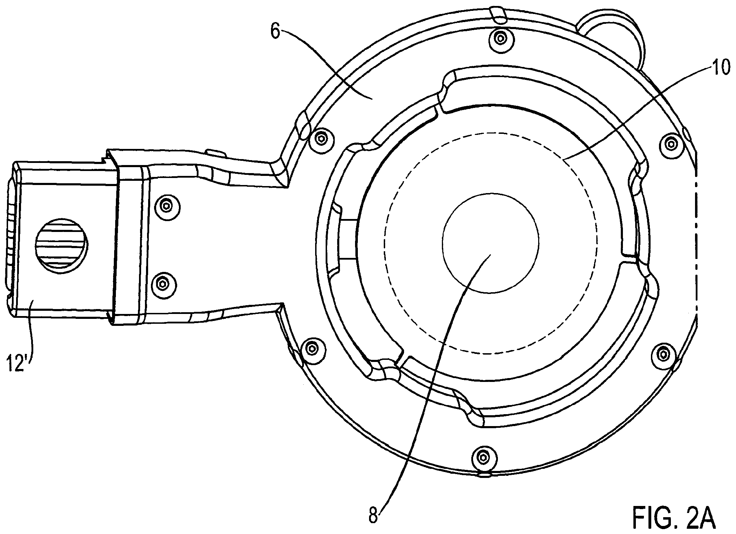

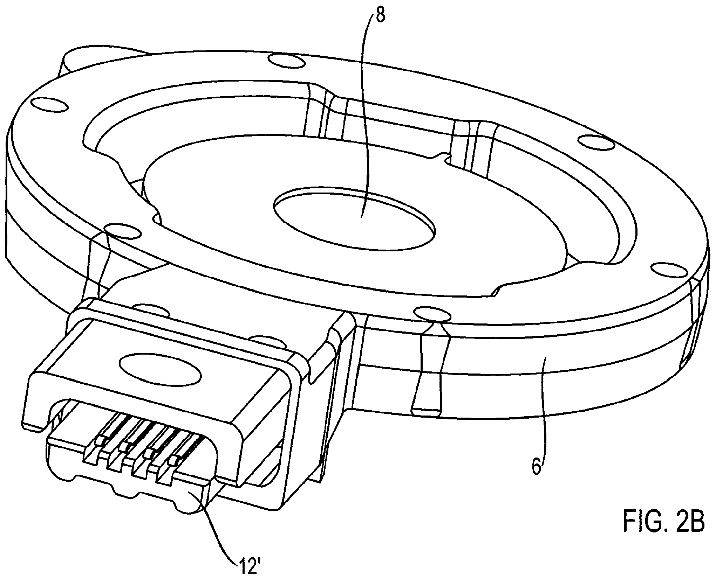

For example, **Figs. 2A** and **2B** respectively show a schematic perspective top view and side view of a vibration head of an aerosol generator including a support member 6, a vibratable membrane 8, and an annular piezoelectric element 10, and a connecting unit 12' for connecting a 4-pin connection cord with the controller. As shown here, the annular piezoelectric element (vibration generating element) 10 has an outer diameter of, for example, about 2 cm. Further, the vibratable membrane 8 and/or the support member 6 has an outer diameter that is larger than the annular piezoelectric element (vibration generating element) 10 and has an inner diameter (corresponding to the inner diameter of the annular piezoelectric element) of, for example, about 1 cm. Within the inner diameter of the vibration generating element 10, the membrane 8 may be dome shaped and comprises the plurality of holes as described above. In addition, the annular piezoelectric element 10 is shown using dotted lines in Fig. 2A indicating that the piezoelectric element 10 is provided at the back side (concave side) of the membrane. In usage, such a vibration head may be placed in an aerosol generator and electrically connected with the external control via the connecting unit 12' using a connection cord. Also here, an appropriate placement of the vibration head in the aerosol generator and a proper electrical connection is decisive for securing a sufficiently high quality of the generated aerosol.

US 2018/313556 A1 discloses a floating humidifier using a vertical power transmission scheme and includes: a wireless power transmitting unit including a substrate and a vertical structure vertically arranged on the substrate, and configured to transmit electric power in a magnetic induction scheme through the substrate and the vertical structure; a water container including an accommodation portion in which water is accommodated and a fastening portion protruding from a center of the accommodation portion and fitted in the vertical structure above the wireless power transmitting unit; and a humidification generating unit inserted into the fastening portion of the water container through a vertically penetrated fastening hole and configured to generate a current using a magnetic force induced by the wireless power transmitting unit to drive a humidifier.

WO 2011/083380 A1 discloses a nebulizer for aerosol delivery of a substance. The nebulizer comprises a mouthpiece, a chamber holding the substance, a flexible membrane having a plurality of apertures, and a vibrator for vibrating the flexible membrane to form a flow of aerosol droplets of the substance that are ejected through the apertures and to a mouthpiece. The vibrator may be a piezoelectric element.

### SUMMARY OF THE INVENTION

### TECHNICAL PROBLEM

An electric connection used for power transfer between the vibration head of the aerosol generator and the controller is conventionally provided via an electric cable having plug connectors. Using an electric plug connector has technical disadvantages due to the fact that a plug-in connector has a limited lifetime being related to a limited number of plug-in operations or plugging cycles, of e.g. 2,000 - 10,000 plug application cycles, that guarantee a secure electrical connection.

In addition, moisture, steam or the like which may be formed as a result of the aerosol generation process or the cleaning process may enter the plug connector and thus leads to leakage currents or even a short circuit between the electric contacts, even in cases in which a sealing is provided. Moisture or high humidity within an electrical contact region (related to the connecting unit 12' in **Figs. 2A** and **2B****,** for example) and/or into a region of the piezoelectric element of the vibration head, may result in a short circuit pre-stage in which the proper electric operation of the vibration head may be impaired. For example, aerosol TOR (total output rate) reduction values of up to 30% have been measured in such situations. In the worst case, ingress of water or a sodium chloride solution may also lead to a complete short circuit in which the operation of the vibration head fails. More specifically, water or liquid ingress may lead to a reduced parallel electrical resistance value of, for example, 1 kS2, compared to an electrical resistance value of the vibration head of, for example, 470 kS2.

Further, a mechanical contact by means, for example, of the plug connector results in regions that are difficult to clean, at least in part.

US 9,962,505 B2 describes a magnetic field (inductive) coupling between a transmitter coil and a receiver coil to electrically drive a vibration source (piezoelectric element). This solution has, however, several disadvantages. First, to achieve a sufficient inductive coupling, a relatively high frequency AC drive current of above 1 MHz is provided through a primary winding of the inductive coupling. Such a high frequency is, however, detrimental to the aerosol quality. Moreover, the spatial maximum dimension of the transmitter coil and the receiver coil in US 9,962,505 B2 as compared to the spatial maximum dimension of the membrane and the piezoelectric element does not allow the integration of such coils into the vibration heads as shown in **Figs. 1A, 1B****,** **2A****,** and **2B****.**

### SOLUTION

Therefore, a need exists in the art for overcoming the above technical problems related to the conventional way of power connecting aerosol generators.

This object is achieved, in a first aspect, by the features of a vibration head for an aerosol generator as defined in claim 1. Advantageous embodiments of the vibration head are described in the corresponding dependent claims.

This object is achieved, in a second aspect, by the features of a system of a controller and an aerosol generator as defined in claim 7. Advantageous embodiments of the system are described in the corresponding dependent claims.

This object is achieved, in a third aspect, by the features of an aerosol generator as defined in claim 18.

### BRIEF DESCRIPTION OF THE DRAWINGS

**Figs. 1A** und **1B** show a schematic perspective bottom view and top view of a vibration head of an aerosol generator, respectively.
**Figs. 2A** and **2B** show a schematic perspective top view and side view of another vibration head of an aerosol generator respectively.
**Fig. 3** schematically shows a vibration head for an aerosol generator and a controller for the aerosol generator according to an embodiment of the present invention.
**Fig. 4** schematically shows a vibration head for an aerosol generator according to another embodiment of the present invention.
**Fig. 5** schematically shows a vibration head for an aerosol generator according to another embodiment of the present invention.
**Fig. 6** schematically shows a vibration head for an aerosol generator and a controller for the aerosol generator according to another embodiment of the present invention.
**Figs. 7A** and **7B** show equivalent electric circuit diagrams for a piezoelectric element and an electric contacting element, respectively.
**Figs. 8A, 8B,** and **8C** respectively show a controller, an aerosol generator, and a connected controller and aerosol generator according to another embodiment of the present invention.

### DESCRIPTION OF THE EMBODIMENTS

Embodiments of the present invention are described with reference to the Figures. It is noted that the following description should not be construed as limiting the invention. In the following and the above, similar or same reference signs indicate similar or same elements or operations.

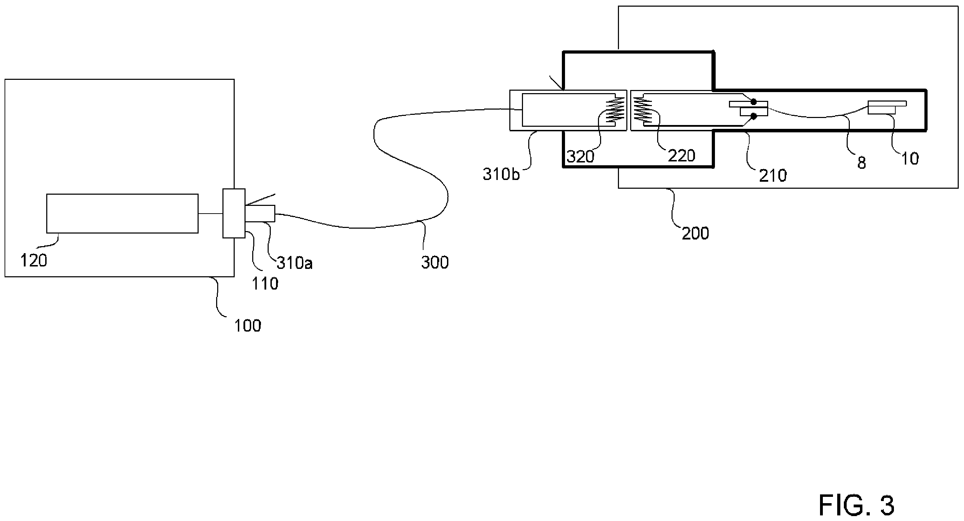

**Fig. 3** schematically shows a controller 100 and a vibration head 210 according to an embodiment of the present invention. The vibration head 210 may be replaceably provided within the aerosol generator 200 and may thus be taken out from the aerosol generator 200, for example for the purpose of cleaning, or may be replaced with another vibration head of the same or a different type. For generating aerosol, the vibration head 210 comprises a membrane 8 and a vibration generating element 10, such as a piezoelectric element, as detailed above.

The aerosol generator 200 has a housing (not shown) with at least a first holing member and a second holding member. The vibration head 210 is at least partially accommodated in the housing and is held between the first and second holding member. For example, the vibration head 210 may be placed with regard to the first holding member and the aerosol generator may be brought into an operating state by closing the second holding member with regard to the first holding member. Further aspects of the mechanical placement, appropriate orientation, and the like of the vibration head 210 within the aerosol generator 200, for example also with regard to a liquid reservoir provided in the aerosol generator are known to the skilled person, see, for example, EP 2 957 349 A1. It is noted that a liquid reservoir is not part of the described vibration head 210. As such, when taking out or detaching the vibration head 210 from the aerosol generator 200, the vibration head 210 may be completely cleaned or disinfected without a further intervention on the vibration head 210, such as opening parts of the vibration head or the like.

As further shown in **Fig. 3****,** the controller 100 comprises a connection unit 110 and a control unit 120.

The connection unit 110 of **Fig. 3** is configured to connect an electric contacting element 300. The electric contacting element 300 may be at least one of an electronic cable, a connector, a connection cord, a print circuit, a circuit path, a conductive polymer, an electric wire, a pin, and a plug.

For simplifying the understanding, **Fig. 3** schematically illustrates a connection cord/electric cable as the electric contacting element 300. As shown, the electric cable has, on one side, a plug-type connection unit (plug connector) 310a, for example having two or more poles for an electric connection with the control unit 120, and has, on the other side of the electric cable 300, a primary coil 320 (schematically shown) within a plug-type connection unit 310b of the electric contacting element (cable) 300 configured for providing an inductive coupling to a secondary coil 220 of the vibration head 210.

In other words, as further schematically illustrated in **Fig. 3****,** the electric contacting element 300 may be provided, on the first side, with a multiple-pin plug 310a or the like in order to provide an electrical connection with the connection unit 110. As shown, the electric contacting element 300 may also be electrically connected, on the second side, with the vibration head 210, e.g. via a plug-type connection unit (plug) 310b or the like, in order to provide a secure electric connection between the controller 100 and the vibration head 210, for example for the purpose of electrically driving the vibration head 210 in accordance with specific electric parameters for generating an aerosol. The skilled person understands that the plug 310b is provided with the primary coil 320 instead of one or more pins as provided in the multiple-pin plug 310a on the first side of the electric contacting element 300. Here, the primary coil 320 preferably has a spatial extension corresponding to the size of the plugs 310a and 310b, so that the plugs 310a and 310b may have substantially the same size. That is, the primary coil 320 may preferably be provided inside a conventionally sized cable plug (plug-type connection unit).

The electric contacting element 300 may therefore be connectable with both the controller 100 and the vibration head 210, or may have at least a fixed connection with the controller and is only connectable with the vibration head 210. In other embodiments, the electric contacting element 300 may be a contacting element providing a contacting mechanism for a direct connection of the controller 100 with the aerosol generator 200 (including the vibration head 210) so that the primary and secondary coils are brought close to each other by the plug-type connection unit shown in Fig. 3.

As further illustrated in **Fig. 3** the vibration head 210 is provided with a secondary coil 220. As illustrated in **Fig. 3****,** the secondary coil 220 is electrically connected with the vibration generating element 10 which may be a piezoelectric element. According to this embodiment, the secondary coil 220 has a spatial dimension that is smaller than an (outer) diameter of the membrane 8 and/or an (outer) diameter of the vibration generating element 10 and may thus be advantageously provided inside the vibration head 210. As the secondary coil 220 is integrated into the vibration head 210 and the vibration head 210 therefore does not have open electrical contacts, the vibration head 210 may be completely cleaned or disinfected when taking out or detaching the vibration head 210 from the aerosol generator 200 without a further intervention on the vibration head 210, such as opening parts of the vibration head or the like. According to this embodiment, also the primary coil 320 has a spatial (maximum) dimension that is smaller than the (outer) diameter of the membrane 8 and/or the (outer) diameter of the vibration generating element 10. As such, the spatial (maximum) dimension of the primary and the secondary coils may be adapted to a plug-type connection unit of the vibration head. In particular, the largest spatial dimension (spatial maximum dimension) of the primary and the secondary coils are such that the coils fit into a plug-type connection unit of the vibration head.

As shown in **Fig. 3****,** when the vibration head 210 is inserted into the aerosol generator 200 and the electric contacting element 300 is plugged into the vibration head 210, then the primary coil 320 within plug 310b is placed in close proximity to the secondary coil 220 of the vibration head, for example at a distance that is a fraction of the coil diameters. In particular, the primary and secondary coils should be placed having a distance of less than 3 millimeter, preferably less than 2 millimeter, and more preferably less than 1 millimeter. The skilled person understands that such a distance may include a plastic casing of the plug 310b and of the vibration head 210. In addition, the plastic casing of the plug 310b and/or of the vibration head 210 may be provided with alignment elements in order to provide an optimized placement of the coils.

Inserting the plug 310b into the vibration head 210 provides a mechanically fixed placement of the primary coil 320 with regard to the secondary coil 220 which, conversely, may be provided in close proximity to a cable connector region.

Such a configuration allows for a sufficient inductive coupling between the primary coil 320 and the secondary coil, and therefore achieves an inductive coupling between the controller 100 and the vibration head 210 for the purpose of power transfer to the aerosol generator 200, in particular at an AC drive current frequency of significantly less than 1 MHz which guarantees an optimal aerosol quality (see below). In particular, an electric excitation of the primary coil 320 having a plurality of windings generates a magnetic flux which acts upon the secondary coil 220 and a voltage is induced in the secondary coil 220. Here, the electric excitation and configuration parameters of the primary and secondary coils are selected in such a way that a sufficient coupling constant is achieved so that a suitable voltage is induced which allows for the vibration generating element 10 to vibrate the membrane 8 to generate an aerosol. Here, the primary and secondary coils preferably have inductance values between 20 to 500 µH which determines the coil material and the number of windings that may be used for the respective plugs.

The inductive coupling is thus provided here for an electric driving (inductive power transfer) of the vibration generating element 10 (piezoelectric element) of the vibration head 210, not for the purpose of charging a battery.

The provision of the vibration head 210 with a mechanically stable mechanism to plug-in and lock the electric contacting element 300 may provide the additional advantage that a lateral offset between the primary and secondary coils is minimized and thus an improved inductive coupling is achieved which minimizes power loss, in particular because an (practically) identical placement of the primary and secondary coils over the lifetime of the plug-in connection may be secured.

In the context of driving the vibration head 210 of the aerosol generator 200, the primary and secondary coils are preferably configured for a transfer of an alternating voltage at 70 Vₚₚ at a frequency of 30 - 180 kHz, more preferably 110 - 170 kHz, at an electrical power of 2 W. As such, the control unit 120 may provide a corresponding electric excitation to the primary coil 320, i.e. an alternating voltage at 70 Vₚₚ at a frequency of 30 - 180 kHz, more preferably 110 - 170 kHz, at an electrical power of 2 W. Surprisingly, the present inventors have found that these electrical parameters may be used by integrating secondary coils into vibration heads, as described above, without changes of the physical dimensions thereof and also without detrimental effects on the aerosol properties, such as TOR (Total Output Rate) and MMD (Mass Median Diameter), in particular at these excitation frequencies (for example between 110 - 170 kHz). In particular, MMD values smaller than 5 µm may be achieved even with the present inductive coupling at these AC drive currents.

In a further embodiment, the secondary coil 220 may be integrated with the vibration head 210, for example within corresponding plastic or synthetic parts of the vibration head 210. As such, when the vibration head 210 is taken out from the aerosol generator 200, for example for the purpose of cleaning, then the secondary coil 220 is removed together with the vibration head 210, while a complete enclosure is provided so that the secondary coil is not damaged when cleaning the aerosol head.

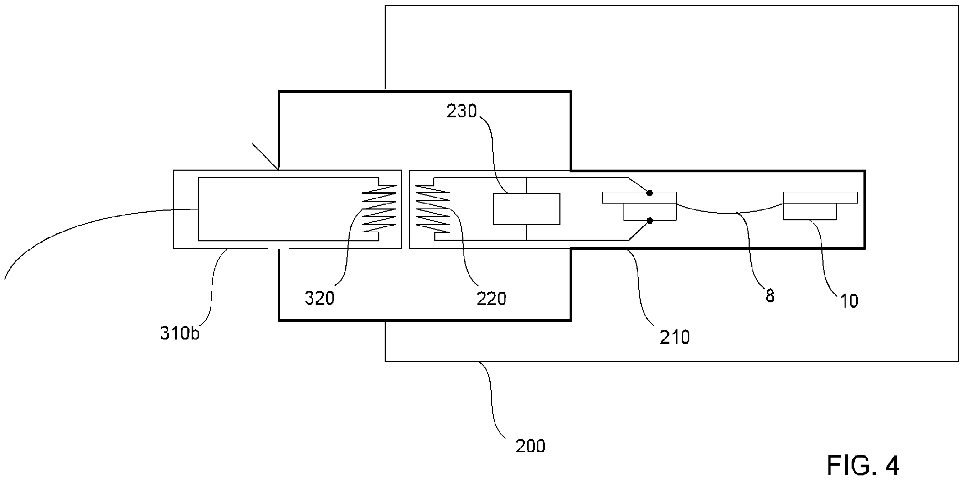

**Fig. 4** shows another preferred embodiment in which the vibration head 210 is further provided with a matching network 230. The matching network 230 has an electrical connection with both the secondary coil 220 and the vibration generating element (piezoelectric element) 10. The matching network 230 is configured for a further adaptation of an electric characteristic of an oscillation circuit comprising the secondary coil 220 and the vibration generating element 10, which may be achieved by providing the matching network 230 with one or more of an electrical conductor, a capacitor, and an inductor. As such, the induced alternating current does not drive the vibration generating (piezoelectric) element 10 directly but the induced voltage is matched to the particular electrical characteristic of the vibration generating element 10. The electrical characteristic may refer, for example, to the resonance frequency of the vibration generating element 10. The matching network 230 is thus adapted to match the induced voltage characteristics to the optimal resonance frequency of the vibration generating element 10 which may thus further improve the aerosol generating efficiency and aerosol quality. Here, the matching network 230 may be provided with one or more inductors of 100 to 200 µH, and one or more capacitors of 100pF to 5000pF, preferably 400 pF to 2000pF. The present embodiment is not limited to providing the matching network within the vibration head. Alternatively, a matching network for optimizing the inductive coupling may also be provided in the controller, i.e. on the side of the primary coil.

Further, by providing the matching network 230 in the vibration head 210, an additional electrical load (in addition to the vibration generating element 10) may by supplied with electric energy, such as one or more sensors which may require different electrical parameters than the vibration generating element 10. In such a case, the primary and secondary coils thus provide a single inductive coupling with regard to at least two different electric loads. This avoids a scenario in which the relatively small space available for the primary and secondary coils would have to be shared with a second inductive coupling due to additional coils for the purpose of providing electric energy to the sensors.

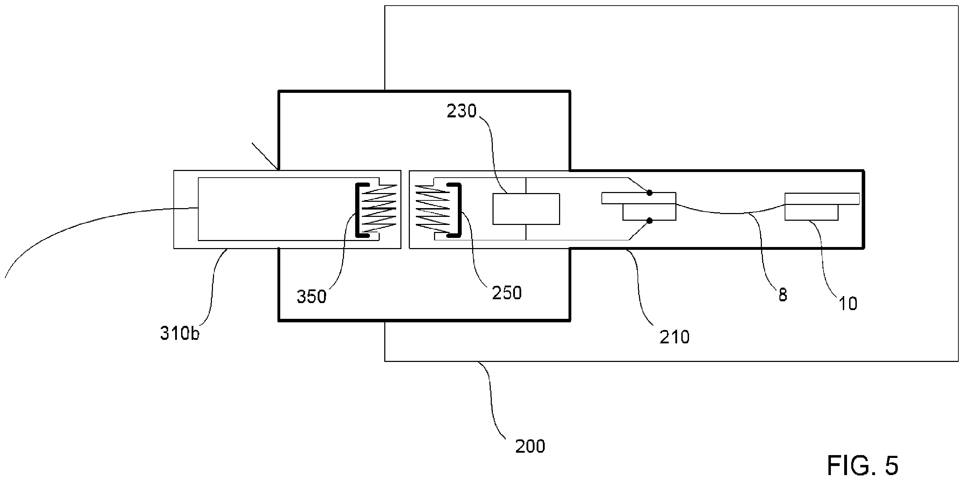

**Fig. 5** shows another preferred embodiment in which the vibration head 210 is further provided with a magnetic field confining element 250 that confines or bundles a magnetic field with regard to the secondary coil 220. This magnetic field confining element 250 may be a magnetically permeable material, such as a ferromagnetic metal, ferrite, or the like, and confines a magnetic field flux emanating from the primary coil 320 in a direction towards the secondary coil 220. The inductive coupling is thus enhanced because the increased magnetic flux at the secondary coil 220 increases the induced voltage and reduces losses. In addition, an electric interference with other components of the aerosol generator, such as a voltage induction at sensors of the aerosol generator, may be reduced, which may also reduce electromagnetic compatibility problems of the medical device (aerosol generator).

As further shown in **Fig. 5****,** also the electric connecting element 300 may be provided with a corresponding magnetic field confining element 350 to confine the magnetic field flux emanating from the primary coil 320 in a direction towards the secondary coil 220. This additional magnetic field confining element 350, which may be provided in the plug 310b, may further enhance the inductive coupling and thus lead to a further reduction in the power demands for driving the vibration head.

Preferably the magnetic field confining elements 250 and 350 may be provided with a pot-shaped core. Typically, the shape of such a pot core is round with an internal hollow that almost completely encloses the coil. Such a pot core may be made by two halves which fit together around a coil former and has a confining effect and prevents radiation and reduces electromagnetic interference. The usage of such pot-shaped cores as magnetic field confining elements 250 and 350 provides a simpler positioning of the primary and secondary coils, in particular in the relatively small space of the plug-type connection units. Preferably, the pot-shaped cores should deviate from each other by less than 1 mm in the cross-area or diameter, even more preferred by less than 0.5 mm, 0.3 mm, or 0.1 mm providing successively improved coupling efficiency.

It is further noted that also a spatial maximum dimension of the magnetic field confining elements 250 and 350 is smaller than a spatial maximum diameter of the membrane 8 and/or the vibration generating element 10. In particular, the spatial dimension of the magnetic field confining elements 250 and 350 related to receive the respective coil windings of the primary and secondary coils, e.g. the spatial dimension of the pot-shaped core is smaller than a diameter of the membrane 8 and/or the vibration generating element 10.

Based on the contactless, inductive coupling provided between the control unit 120 and the vibration head 210, the controller 100 and the vibration head 210 are separate structural components and may be provided with a complete enclosure, i.e. no open electrical contacts. In particular, each component may be provided with a separate casing to avoid moisture/water entry. In addition, this allows for an improved chemical and/or thermal disinfection, for example because the disinfection does not affect the moulded coils as compared to the open electric contacts of the conventional system that leads to corrosion. In particular, a synthetic material that may be used for overmolding the coils is resistant to temperatures of 100°C and above. The positioning of the coils for inductive coupling may thus be maintained without deformation.

In a further embodiment, the contactless, inductive coupling may further be used for the additional transmission of information data, for example for the purpose of transmitting control parameter/inhalation data to/from the vibration head 210. For this, a transmission-side unit at the controller 100 and/or the vibration head 210 appropriately modulates the information data onto the excitation signal (described above) and a corresponding reception-side unit at the controller 100 and/or the vibration head 210 filters/demodulates this excitation signal. Here, the information data may refer to sensor and/or storage data, for example to drive sensors or actuators or to read out storage areas of the sensors at the aerosol generator or at the vibration head.

An example of such a sensor is a fluid presence sensor for detecting whether a sufficient amount of fluid/liquid is present in the reservoir. Another example of such a sensor is a flow sensor for detecting whether a patient/user is inhaling or exhaling. As illustrated above, one or more such sensors may be supplied with electric energy via the single inductive coupling provided between the primary and secondary coil, preferably in addition to enabling data transmission.

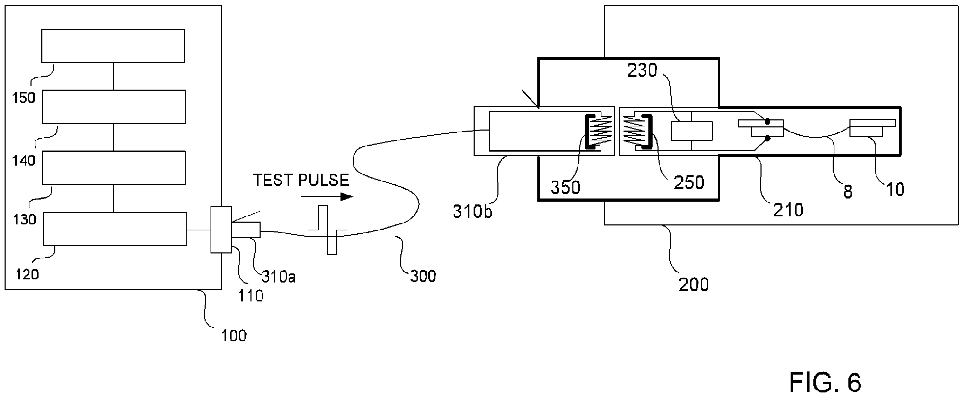

In a further embodiment illustrated in **Fig. 6** the controller 100 is configured to determine whether an inductive coupling based vibration head 210 of an aerosol generator 200, as described above, is properly connected. In other words, the controller 100 is configured to detect whether a vibration head 210 is electrically connected via the above described inductive coupling and an aerosol generation is thus possible.

As illustrated in **Fig. 6****,** the controller 100 further has a detection/determination unit 130, a notification unit 140, and a storage unit 150.

The detection/determination unit 130 is configured to detect an energy storage parameter related to the connected electric contacting element 300 and to determine whether the vibration head 210 is connected with the electric contacting element 300 based on a determined energy storage parameter.

Such a detection and determination mechanism may be of particular interest for patients in intensive medical care, for which a medication or drug administration is provided via a respiratory device, and which themselves are not capable of verifying the proper/correct aerosolization of the medication or drug. In addition, such an intensive medical care setup may require the presence of rather long electrical cables that connect the controller 100 with the aerosol generator 200 (being provided with an inserted inductive coupling based vibration head 210) and which are thus prone to disconnection errors. Therefore, a determination as to a correct connection of the vibration head 210 minimizes errors in medical treatments. Such a detection and determination mechanism may also be of particular interest in situations in which the controller 100 is in a remote position from the aerosol generator (being provided with an inserted inductive coupling based vibration head 210). In such a situation, the proper connection may, for example, not be visually checked.

In the following, two cases are distinguished. In a first case, the electric contacting element 300 is connected *only* with the connection unit 110 of the controller 100, while in a second case the electric contacting element 300 is connected with both the connection unit 110 of the controller 100 and the vibration head 210 is properly placed into the aerosol generator 200 and the plug 330 of the electric contacting element 300 is mechanically connected with the vibration head 210 so that an inductive coupling between the primary coil 320 and the secondary coil 220 is efficiently achieved. Only in the second case, the vibration head 210 has a contactless electric connection with the controller 100 and a proper aerosol generation can be ensured.

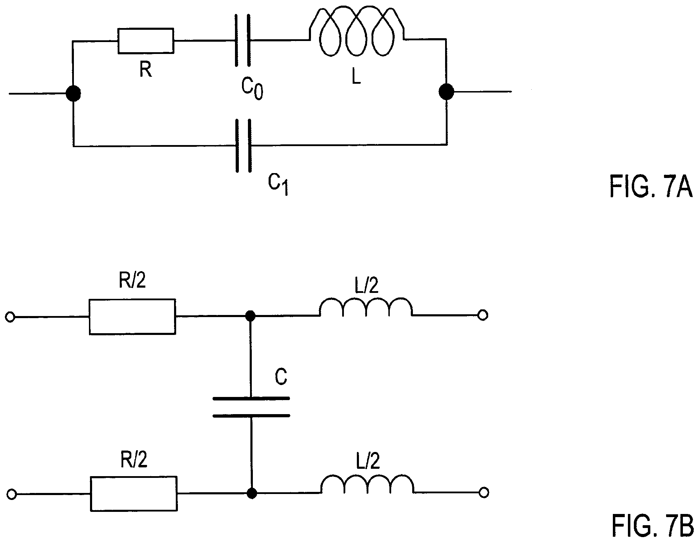

The present inventors have realized that the controller 100 itself is able to distinguish between these two cases on the basis of an energy storage parameter. Here, the energy storage parameter is associated or related to the connected electric contacting element 300 in the above first case, and is associated or related to the connected electric contacting element 300 and the connected vibration head 210 combined in the above second case. The energy storage parameter refers to at least one of capacity (electric charge storage in a capacitor) and inductance (magnetic field energy) that may be (temporarily) stored in the vibration head 210 and the electric contacting element 300, respectively. **Figs. 7A** and **7B** show equivalent electric circuit diagrams of the inductive-based vibration head (including the piezoelectric element 10 and the secondary coil 220) (**Fig. 7A**) and an electric cable (including the primary coil 320) as an example of the electric contacting element 300 (**Fig. 7B**). Here, the piezoelectric element of the vibration head 210 may be considered as having an electric energy storage capability due to capacitive (C₀, C₁) circuit elements. Further, the secondary coil 220 of the vibration head 210 may be considered as having a magnetic field energy storage capability due to inductive (L) circuit elements in **Fig. 7A****.** Also, the electric cable may be considered as having an energy storage capability due to the capacitive (C) and inductive (L/2, L/2) circuit elements in **Fig. 7B****.** Here, the inductive circuit elements of the electric cable refer to an inductance value of the primary coil 320.

The energy storage type of a piezoelectric element in a vibration head 210 may be primarily of capacitive nature with values of 4 - 7 nF, while the primary/secondary coil typically has inductance values from 20 to 10.000 µH. The storage type of an electric cable may be primarily of capacitive nature with values of 0.05 - 0.25 nF. In other words, both the vibration head 210 and the electric contacting element 300 have distinguishable energy storage parameters.

Based on the above, in the above first case, the energy storage capacity related to the connected electric contacting element 300 is thus the energy storage capacity of the electric contacting element 300. In the above second case, i.e. when also the vibration head 210 is connected due to inductive coupling, then the energy storage capacity related to the connected electric contacting element 300 is the energy storage capacity of both the electric contacting element 300 and the vibration head 210, i.e. the amount of electric energy (electric field and/or magnetic field) that may be stored in both the electric contacting element 300 and the vibration head 210.

As will be further detailed below, the detection of a parameter related to the energy storage capacity may be performed by a pulsed measurement, for example by a mono-polar or bi-polar electric pulse measurement. This may be considered as a test pulse measurement or a pre-phase measurement. The mono-polar test pulse may be provided by a simple pulse generating set up, while a bi-polar test pulse is advantageous in order to electrically discharge the piezoelectric element 10 of the vibration head 210 completely. Here, responsive to applying an electrical test pulse to the connected electric contacting element 300, an electric discharge profile, i.e. an electric discharge curve over a defined electric load in the first case (only electric contacting element connected) or second case (electric contacting element and inductive coupling based vibration head connected), may be detected by the detection unit 120. Such electric discharge curves may be a voltage discharge profile typically having an exponential temporal decay U(t) - U0 × exp(-t/τ) in which U is a measured voltage, t is time, and τ is an example of an energy storage parameter that indicates a corresponding capacitance and/or inductance value of the connected load. In the above first case in which only the electric contacting element 300 is connected, the energy storage parameter τ1 is thus a value that is typically much smaller than in the above second case in which also the inductive coupling based vibration head 210 is connected and the energy storage parameter is τ2, i.e. τ1 < τ2.

More specifically, the detection/determination unit 130 may thus be configured to analyze the measured discharge curve and determine an energy storage parameter therefrom. Here, the analysis of the measured discharge curve may be performed by the detection/determination unit 130 by applying one or more fitting parameters to the measured discharge curve. Based on the determined energy storage parameter (τ1, τ2), the detection/determination unit 130 thus determines whether the inductive coupling based vibration head 210 is correctly connected (τ ≅ τ2) or whether the inductive coupling based vibration head 210 is not connected (τ ≅ τ1). Thus, determination may be performed by comparing the detected energy storage parameter with a predetermined threshold. Such a predetermined threshold may be set on the basis of typical values τ1 for the electrical contacting element 300. It is noted that different electrical contacting elements (electronic cable, a connector, a print circuit, a circuit path, a conductive polymer, an electric wire, a pin, a plug, or a combination thereof) may be used in practical applications, but that the energy storage parameter of an inductive coupling based vibration head is always significantly larger, as explained above.

The notification unit 140 is configured to notify a user, via at least one of a visual, audio, or a haptic feedback, of a determination result of the detection/determination unit 130. For example, if an inductive coupling based vibration head 210 is connected a green light may be shown to thus user, while a red (e.g., blinking) light is shown to the user if the determination result indicates that an inductive coupling based vibration head 210 is not connected. In particular, the notification may provide a warning that an inductive coupling based vibration head is not connected with the controller 100 and, therefore, the aerosolization is not started and an aerosol generation cannot be accomplished. This is, for example, helpful in hospital environments in which rather long electric cables 300 are used and the proper electric connection between the controller 100 and the vibration head 210 may be lost due to obstacles or the like. Alternatively, or in addition, the notification unit 140 may send a communication message, e.g. an e-mail, SMS, warning signal, or the like, to a third party (e.g. a central hospital monitoring system or the like).

The above measurement may be performed during the regular activation of the inductive coupling based vibration head 210, i.e. an electric activation of the inductive coupling based vibration head 210 when an aerosol is generated (online operation), and may also be performed in a pre-phase (i.e. a starting phase) of the aerosol generation activation, or during a stand-by mode in which no aerosol is generated. During the stand-by operation, for example, a pulsed electric excitation of the inductive coupling based vibration head (via the electric contacting element 300) uses electric parameters (voltage, current) which are not sufficient to vibrate the vibration head 210 in a way to generate aerosol. Such a stand-by measurement therefore avoids an actual aerosol generation and associated drug loss that would result from an operation of the aerosol generator during an on-line operation in which the inductive coupling based vibration head is supplied with alternating current. In this way, no liquid medication is wasted when it is determined (only) that a proper electric connection of the inductive coupling based vibration head 210 is achieved.

In another embodiment, the detection/determination unit 130 may be further configured to determine a vibration head type. As discussed above, different inductive coupling based vibration head types may be provided for the aerosol generator 200. In particular, different electrical excitation parameters may be required for the different inductive coupling based vibration head types so that the determination of the connected vibration head type enables the controller 100 to select and apply corresponding control and/or drive parameters (voltage, current, and/or frequency).

The detection/determination unit 130 may distinguish between a plurality of different inductive coupling based vibration head types on the basis of the detected energy storage parameter. For each connected inductive coupling based vibration head type a distinguishable electric discharge curve can be obtained. As such, the detection/determination unit 130 may be provided with respective ranges for the energy storage parameter that are vibration head specific. In other words, these ranges may be specific for different inductive coupling based vibration heads to uniquely identify the respective vibration head type. For example, for a Type 1 vibration head, a specific parameter range between a minimal (min) and maximum (max) value may be provided, i.e. τ2 (min) ≤ τ2 ≤ τ2(max), and a specific parameter range may also be provided for a Type 2 vibration head, i.e. τ2(min) ≤ τ2 ≤ τ2(max). The detection/determination unit 130 may then determine the connected inductive coupling based vibration head 210 by comparing the detected energy storage parameter τ2 with these ranges.

The storage unit 150 of **Fig. 6** may also store a plurality of vibration head type dependent configuration parameters. The configuration parameters may refer to head-type dependent electric parameters (voltage, current, and/or frequency) for operating the inductive coupling based vibration head. Then, the control unit 120 of **Fig. 6** may select, based on the determined vibration head type, one of the vibration head type dependent configuration parameters and control the inductive coupling based vibration head 210 of the aerosol generator 200 to generate aerosol based on the selected vibration head type dependent configuration parameter. As such, the appropriate electrical drive parameters are used for generating the aerosol and an appropriate aerosol quality may be ensured. In addition, a single controller 100 may operate with a variety of different inductive coupling based vibration head types, and it is therefore no longer required to provide respective controllers for each of the different inductive coupling based vibration head types.

Based on the above, a cost-efficient way to determine whether an inductive coupling based vibration head is connected with the controller of the aerosol generator is provided. The controller may be used, without further programming, with regard to different vibration head types, and thus simplifies the usability. In addition, an independent signal connection as well as an independent identification device (characterization device) for the purpose of identifying the vibration head type, as shown in US 8,720,432 B2, for example, may be omitted.

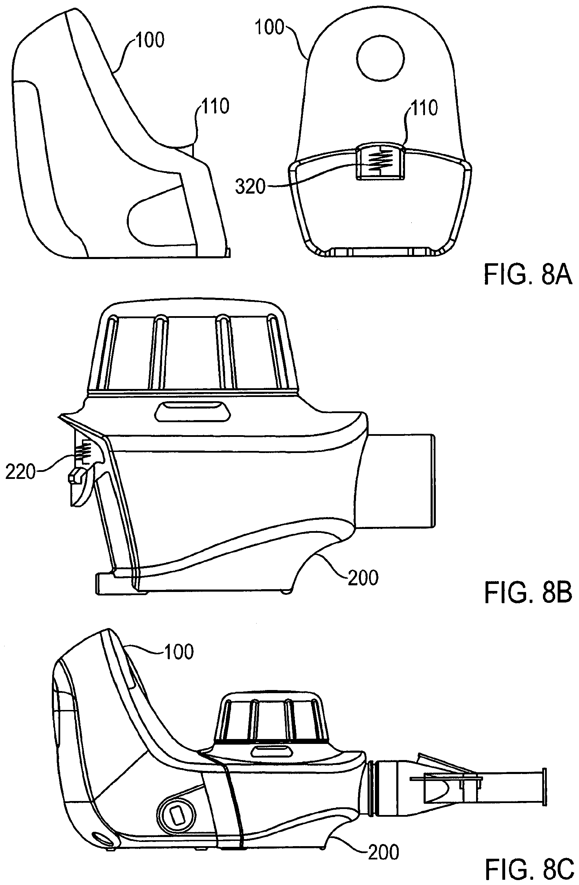

**Figs. 8A, 8B,** and **8C** respectively show a controller 100 (side view and front view), an aerosol generator 200, and a connected controller 100 and aerosol generator 200 according to another embodiment of the present invention. As indicated in **Fig. 8A****,** a primary coil 320 may be directly provided at the controller, in particular within the connection unit 110. Likewise, in **Fig. 8B****,** the secondary coil 220 is schematically shown to be provided at the vibration head (not shown) of the aerosol generator 200. In **Fig. 8C****,** the controller 100 and the aerosol generator 200 are plugged together, so that the primary coil 320 and the secondary coil 220 are brought close to each other and an inductive coupling is achieved, as explained above. That is, the electric contacting element is here provided by a plug-type contacting mechanism (without an extra cable, or the like) for a direct connection of the primary and secondary coils. It is further noted, that the primary and secondary coils are not exposed to the outside (no open electrical contacts) with this contacting mechanism, but may be overmolded by a synthetic material, as explained above.

According to a further embodiment, the control unit 120 of the controller 100 may be configured to alternatingly generate excitation signals of at least two different frequencies. Here, the excitation signal of a first frequency (f₁) may be the above described excitation signal which is supplied via the contactless inductive coupling in the vibration head 210 to the vibration generating element 10 in order to cause the membrane 8 to oscillate/vibrate and to generate the aerosol. The excitation signal of the second frequency (f₂), on the other hand, may be related to a frequency that is used for determining an operating state of the vibration head 210. The time periods in which the second frequency signal is supplied to the vibration generating element 10 via the inductive coupling, provided by the primary coil 320 and the secondary coil 220, are typically much shorter than the time periods in which the first frequency signal is supplied. This is because the second frequency signal is supplied for measuring purposes only and should prevent a disturbance or interruption of the generation of the aerosol.

Here, the operating state of the vibration head 210 may refer to respective specific characteristics during nebulization and during an operation without a liquid, i.e. operating states with and without a liquid on the vibrating membrane 8. By detecting an electric parameter of the vibration head, such as electric current, voltage, electric power, phase shift, which are dependent on the capacity of the vibration generating element 10, the present inventors have surprisingly found that the operating states with and without liquid on the membrane can also be reliably determined when an inductive coupling to the vibration head 210 is provided, as described above, and the influence of the inductance of the primary and secondary coils cannot be ignored.

In order to detect at least one of the parameters of the vibration head 210, the detection/determination unit 130 of the controller 100 is configured in such a way that, during operation of the control unit 120 and responsive to the above described alternating excitation signals of at least two different frequencies, the at least one electric parameter is tapped from respective connecting lines of the electric contacting element 300 that supplies the excitation signals to the primary coil 320. That is, parameters of the vibration head 210 are inferred by detecting one or more electric parameters related to the primary coil 320.

A determination of the operating state, i.e. a determination of whether liquid is present or not, may be performed in the detection/determination unit 130 by comparing the detected value of the at least one parameter with a value for this parameter stored in the storage unit 150 of the controller 100. It is noted that the stored parameters are parameters that have been predetermined for the specific case of inductive coupling based vibration heads.

Detecting the at least one electric parameter of the vibration head to determine the presence of a liquid to be nebulized is thus even possible when providing inductive coupling based vibration heads.

If, by comparing a detected parameter with a stored parameter, the detection/determination unit 130 determines that there is no more liquid in the liquid reservoir, then, in a preferred embodiment, a corresponding notification signal is sent to the control unit 120 and to the notification unit 140. Upon reception of this notification signal, the control unit 120 automatically and immediately stops the supply of excitation signals to the primary coil, i.e. automatically switches off the aerosol generator 200. Further, the notification unit 140 provides a feedback (at least one of a visual, audio, or a haptic feedback, as described above) to indicate to the user that the aerosol generator has consumed the stored liquid.

In a further embodiment, the detection/determination unit 130 may also determine a breathing state of a user based on one or more electric parameters that are detected with regard to the primary coil 320 (e.g., voltage tap, current consumption, current/voltage phase position at the primary coil). This embodiment follows the observation that pressure fluctuations during breathing (i.e. during inhalation and during exhalation) act upon the vibrating membrane 8 and are sufficiently large to lead to an output signal that is emitted by the vibration generating piezoelectric element 10 and may be also transferred via the inductive coupling to the primary coil where such an output signal may be detected. The detection characteristic may be improved by further providing the detection/determination unit 130 with a low-pass filter unit and an amplifier unit (not shown). As such, a conventional flow sensor may not be necessary simplifying the aerosol generator and reducing the manufacturing costs.

## Claims

1. Vibration head (210) for an aerosol generator (200), the vibration head (210) comprising:
a membrane (8) and
a vibration generating element (10);
**characterised in that** the vibration head further comprises a secondary coil (220) configured for providing an inductive coupling with a primary coil (320) being connected with a controller (100) of the aerosol generator (200), the inductive coupling driving the vibration generating element (10) to vibrate the membrane (8) for generating the aerosol; and
a matching network (230) in connection with the secondary coil (220) and the vibration generating element (10),
wherein the matching network (230) is configured for adapting an electric characteristic of an oscillation circuit comprising the secondary coil (220) and the vibration generating element (10).

2. The vibration head (210) of claim 1, wherein the secondary coil (220) has a spatial maximum dimension being smaller than a spatial maximum diameter of said membrane (8), and/or said vibration generating element (10).

3. The vibration head (210) of claim 2, wherein the spatial maximum dimension of said secondary coil (220) is adapted to a plug-type connection unit of the vibration head.

4. The vibration head (210) of one of claims 1 - 3, wherein the matching network (230) comprises one or more of an electrical resistor, a capacitor, and/or an inductor.

5. The vibration head (210) of one of claims 1 - 4, wherein the secondary coil (220) is integrated within the vibration head (210) and/or
wherein the secondary coil (220) is provided in proximity to a cable connector region.

6. The vibration head (210) of one of claims 1 - 5, further comprising a magnetic field confining element (250) for confining a magnetic field with regard to the secondary coil (220).

7. System comprising a controller (100) and an aerosol generator (200) having a vibration head (210) according to one of claims 1 - 6, the controller comprising:
a control unit (120);
a primary coil (320) configured for providing an inductive coupling to the secondary coil (220) of the vibration head (210), the inductive coupling driving the vibration generating element (10) of the vibration head (210),
wherein the control unit (120) is configured to provide an electric excitation of the primary coil (320).

8. The system according to claim 7, wherein the primary coil (320) has a spatial maximum dimension being smaller than a spatial maximum diameter of said vibration generating element (10).

9. The system according to claim 8, wherein the spatial maximum dimension of said primary coil (320) is adapted to a plug-type connection unit of the controller.

10. The system according to any of claims 7 - 9, further comprising an electric connecting element (300), wherein the primary coil (320) is provided on the electric connecting element (300) connected with the control unit (120) of the controller.

11. The system according to claim 10, wherein said electric connecting element (300) is an electric cable having on one side, a plug connector (310) for connecting with the control unit (120), and having, on the other side of the electric cable, the primary coil (320) configured for providing an inductive coupling to the secondary coil (220) of the vibration head (210).

12. The system according to one of claims 7 - 11, wherein a magnetic field confining element (350) is further provided for confining a magnetic field with regard to the primary coil (320).

13. The system according to one of claims 7 - 12, further comprising;
a detection/determination unit (130) for detecting an electric parameter related to the primary coil (320) and determining an operating state of the vibration head (210) and/or a breathing state of a user based on the detected electric parameter.

14. The system Controller (100) according to one of claims 7
- 12, further comprising;
a connection unit (110) for connecting an electric contacting element (300), the electric contacting element being connectable with the vibration head (210);
a detection/determination unit (130) for detecting an energy storage parameter related to the connected electric contacting element (300) and determining whether the vibration head (210) has an inductive coupling with the electric contacting element (300) based on the energy storage parameter.

15. The system according to claim 14, wherein the detection/determination unit (130) is configured to determine that the vibration head (210) has the inductive coupling with the electric contacting element (300) when the detected energy storage parameter exceeds a predetermined threshold.

16. The system according to one of claims 14 - 15, wherein the detection/determination unit (130) is further configured to determine a vibration head type by comparing the detected energy storage parameter with a plurality of energy storage parameter ranges.

17. The system according to claim 16, further comprising:
a storage unit (150) for storing a plurality of vibration head type dependent configuration parameters;
wherein the control unit (120) is further configured to select, based on the determined vibration head type, one of the stored vibration head type dependent configuration parameters, and to control the vibration head (210) of the aerosol generator (200) to generate aerosol based on the selected vibration head type dependent configuration parameter.

18. An aerosol generator (200), comprising:
a housing, and a vibration head (210) according to one of claims 1 - 6;
wherein the vibration head (210) is at least partially accommodated in the housing and the housing further includes a fluid reservoir.

## Patentansprüche

1. Vibrationskopf (210) für einen Aerosolerzeuger (200), der Vibrationskopf (210) umfassend:
eine Membran (8); und
ein Vibrationserzeugungselement (10);
**dadurch gekennzeichnet, dass** der Vibrationskopf ferner umfasst:
eine Sekundärspule (220), die konfiguriert ist zum Bereitstellen einer induktiven Kopplung mit einer Primärspule (320), die mit einer Steuerung (100) des Aerosolerzeugers (200) verbunden ist, wobei die induktive Kopplung das Vibrationserzeugungselement (10) zum Vibrieren der Membran (8) zur Erzeugung des Aerosols antreibt; und
ein Anpassungsnetzwerk (230) in Verbindung mit der Sekundärspule (220) und dem Vibrationserzeugungselement (10),
wobei das Anpassungsnetzwerk (230) konfiguriert ist zum Anpassen einer elektrischen Eigenschaft eines Schwingungsschaltkreises, der die Sekundärspule (220) und das Vibrationserzeugungselement (10) umfasst.

2. Vibrationskopf (210) nach Anspruch 1, wobei die Sekundärspule (220) eine räumliche Maximal-Abmessung aufweist, die kleiner ist als ein räumlicher Maximal-Durchmesser der Membran (8), und/oder des Vibrationserzeugungselements (10).

3. Vibrationskopf (210) nach Anspruch 2, wobei die räumliche Maximal-Abmessung der Sekundärspule (220) an einer steckerartigen Verbindungseinheit des Vibrationskopfes angepasst ist.

4. Vibrationskopf (210) nach einem der Ansprüche 1 - 3, wobei das Anpassungsnetzwerk (230) einen oder mehrere elektrische Widerstände, einen Kondensator und/oder eine Spule umfasst.

5. Vibrationskopf (210) nach einem der Ansprüche 1 - 4, wobei die Sekundärspule (220) in den Vibrationskopf (210) integriert ist und/oder
wobei die Sekundärspule (220) in der Nähe eines Kabelanschlussbereichs bereitgestellt ist.

6. Vibrationskopf (210) nach einem der Ansprüche 1 - 5, ferner umfassend ein Magnetfeldbegrenzungselement (250) zur Begrenzung eines Magnetfeldes in Bezug auf die Sekundärspule (220) .

7. System, das eine Steuerung (100) und einen Aerosolerzeuger (200) mit einem Vibrationskopf (210) gemäß einem der Ansprüche 1 - 6 umfasst, die Steuerung umfassend:
eine Steuereinheit (120);
eine Primärspule (320), die konfiguriert ist zum Bereitstellen einer induktiven Kopplung mit der Sekundärspule (220) des Vibrationskopfes (210), wobei die induktive Kopplung das Vibrationserzeugungselement (10) des Vibrationskopfes (210) antreibt,
wobei die Steuereinheit (120) konfiguriert ist zum Bereitstellen einer elektrischen Anregung der Primärspule (320) .

8. System gemäß Anspruch 7, wobei die Primärspule (320) eine räumliche Maximal-Abmessung aufweist, die kleiner ist als ein räumlicher Maximal-Durchmesser des Vibrationserzeugungselements (10).

9. System gemäß Anspruch 8, wobei die räumliche Maximal-Abmessung der Primärspule (320) an einer steckerartigen Verbindungseinheit der Steuerung angepasst ist.

10. System gemäß einem der Ansprüche 7 - 9, ferner umfassend ein elektrisches Verbindungselement (300), wobei die Primärspule (320) auf dem elektrischen Verbindungselement (300) bereitgestellt ist, das mit der Steuereinheit (120) der Steuerung verbunden ist.

11. System gemäß Anspruch 10, wobei das elektrische Verbindungselement (300) ein elektrisches Kabel ist, das auf einer Seite eine Steckverbindung (310) zum Verbinden mit der Steuereinheit (120) aufweist und auf der anderen Seite des elektrischen Kabels die Primärspule (320) aufweist, die konfiguriert ist zum Bereitstellen einer induktiven Kopplung mit der Sekundärspule (220) des Vibrationskopfes (210).

12. System gemäß einem der Ansprüche 7 - 11, wobei ferner ein Magnetfeldbegrenzungselement (350) zum Begrenzen eines Magnetfelds in Bezug auf die Primärspule (320) bereitgestellt wird.

13. System gemäß einem der Ansprüche 7 - 12, ferner umfassend:
eine Detektions-/Bestimmungseinheit (130) zum Detektieren eines elektrischen Parameters in Bezug auf die Primärspule (320) und zum Bestimmen eines Betriebszustands des Vibrationskopfes (210) und/oder eines Atemzustands eines Benutzers auf Grundlage des detektierten elektrischen Parameters.

14. Systemsteuerung (100) gemäß einem der Ansprüche 7 - 12, ferner umfassend:
eine Verbindungseinheit (110) zum Verbinden eines elektrischen Kontaktelements (300), wobei das elektrische Kontaktelement mit dem Vibrationskopf (210) verbindbar ist;
eine Detektions-/Bestimmungseinheit (130) zum Detektieren eines Energiespeicherparameters in Bezug auf das verbundene elektrische Kontaktelement (300) und zum Bestimmen, ob der Vibrationskopf (210) auf Grundlage des Energiespeicherparameters eine induktive Kopplung mit dem elektrischen Kontaktelement (300) aufweist.

15. System gemäß Anspruch 14, wobei die Detektions-/Bestimmungseinheit (130) konfiguriert ist zum Bestimmen, dass der Vibrationskopf (210) die induktive Kopplung mit dem elektrischen Kontaktelement (300) aufweist, wenn der erfasste Energiespeicherparameter einen vorbestimmten Schwellwert überschreitet.

16. System gemäß einem der Ansprüche 14 - 15, wobei die Detektions-/Bestimmungseinheit (130) ferner konfiguriert ist zum Bestimmen einer Vibrationskopf-Art durch Vergleichen des detektierten Energiespeicherparameters mit einer Vielzahl von Energiespeicherparameterbereichen.

17. System gemäß Anspruch 16, ferner umfassend:
eine Speichereinheit (150) zum Speichern einer Vielzahl von Konfigurationsparametern, die von der Vibrationskopfs-Art abhängen;
wobei die Steuereinheit (120) ferner konfiguriert ist zum Auswählen eines der gespeicherten, von der Vibrationskopfs-Art abhängigen Konfigurationsparameter auf Grundlage der bestimmten Vibrationskopf-Art und zum Steuern des Vibrationskopfs (210) des Aerosolerzeugers (200), um Aerosol auf Grundlage des ausgewählten, von der Vibrationskopf-Art abhängigen Konfigurationsparameters zu erzeugen.

18. Ein Aerosolerzeuger (200), umfassend:
ein Gehäuse, und
einen Vibrationskopf (210) gemäß einem der Ansprüche 1 - 6;
wobei der Vibrationskopf (210) zumindest teilweise in dem Gehäuse untergebracht ist und das Gehäuse außerdem ein Flüssigkeitsreservoir enthält.

## Revendications

1. Une tête de vibration (210) pour un générateur d'aérosol (200), la tête de vibration (210) comprenant :
une membrane (8) ; et
un élément générateur de vibration (10) ;
**caractérisé en ce que** la tête de vibration comprend également une bobine secondaire (220) configurée pour fournir un couplage inductif avec une bobine primaire (320) connectée avec un contrôleur (100) du générateur d'aérosol (200), le couplage inductif commandant l'élément générateur de vibration (10) pour faire vibrer la membrane (8) pour générer l'aérosol ; et
un circuit d'adaptation (230) en connexion avec la bobine secondaire (220) et l'élément générateur de vibration (10),
où le circuit d'adaptation (230) est configuré pour adapter une caractéristique électrique d'un circuit d'oscillation comprenant la bobine secondaire (220) et l'élément générateur de vibration (10).

2. La tête de vibration (210) selon la revendication 1, où la bobine secondaire (220) a une dimension spatiale maximale étant plus petite qu'un diamètre spatial maximal de ladite membrane (8), et/ou que dudit élément générateur de vibration (10).

3. La tête de vibration (210) selon la revendication 2, où la dimension spatiale maximale de ladite bobine secondaire (220) est adaptée à une unité de connexion à fiche de la tête de vibration.

4. La tête de vibration (210) selon l'une des revendications 1 à 3, où le circuit d'adaptation (230) comprend un ou plus d'une résistance électrique, d'un condensateur, et/ou d'un inducteur.

5. La tête de vibration (210) selon l'une des revendications 1 à 4, où la bobine secondaire (220) est intégrée dans la tête de vibration (210) et/ou où la bobine secondaire (220) est fournie à proximité d'une zone de connexion de câble.

6. La tête de vibration (210) selon l'une des revendications 1 à 5, comprenant de plus un élément de confinement de champ magnétique (250) pour confiner un champ magnétique en regard de la bobine secondaire (220) .

7. Un système comprenant un contrôleur (100) et un générateur d'aérosol (200) ayant une tête de vibration (210) selon l'une des revendications 1 à 6, le contrôleur comprenant :
une unité de contrôle (120) ;
une bobine primaire (320) configurée pour fournir un couplage inductif à la bobine secondaire (220) de la tête de vibration (210), le couplage inductif commandant l'élément générateur de vibration (10) de la tête de vibration (210),
où l'unité de contrôle (120) est configurée pour fournir une excitation électrique de la bobine primaire (320).

8. Le système selon la revendication 7, où la bobine primaire (320) a une dimension spatiale maximale étant plus petite qu'un diamètre spatial maximal dudit élément générateur de vibration (10).

9. Le système selon la revendication 8, où la dimension spatiale maximale de ladite bobine primaire (320) est adaptée à une unité de connexion à fiche du contrôleur.

10. Le système selon l'une des revendications 7 à 9, comprenant de plus un élément de connexion électrique (300), où la bobine primaire (320) est fournie sur l'élément de connexion électrique (300) connecté avec l'unité de contrôle (120) du contrôleur.

11. Le système selon la revendication 10, où ledit élément de connexion électrique (300) est un câble électrique ayant d'un côté, une fiche de connexion (310) pour se connecter avec l'unité de contrôle (120), et ayant, de l'autre côté du câble électrique, la bobine primaire (320) configurée pour fournir un couplage inductif à la bobine secondaire (220) de la tête de vibration (210).

12. Le système selon l'une des revendications 7 à 11, où un élément de confinement de champ magnétique (350) est de plus fourni pour confiner un champ magnétique en regard de la bobine primaire (320).

13. Le système selon l'une des revendications 7 à 12, comprenant de plus ;
une unité de détection/détermination (130) pour détecter un paramètre électrique en rapport avec la bobine primaire (320) et déterminer un état de fonctionnement de la tête de vibration (210) et/ou un état de respiration d'un utilisateur basé sur le paramètre électrique détecté.

14. Le contrôleur de système (100) selon l'une des revendications 7 à 12, comprenant de plus :
une unité de connexion (110) pour connecter un élément de contact électrique (300), l'élément de contact électrique étant connectable avec la tête de vibration (210) ;
une unité de détection/détermination (130) pour détecter un paramètre de stockage d'énergie en rapport avec l'élément de contact électrique connecté (300) et déterminer si la tête de vibration (210) a un couplage inductif avec l'élément de contact électrique (300) basé sur le paramètre de stockage d'énergie.

15. Le système selon la revendication 14, où l'unité de détection/détermination (130) est configurée pour déterminer que la tête de vibration (210) a le couplage inductif avec l'élément de contact électrique (300) quand le paramètre de stockage d'énergie détecté excède un seuil prédéterminé.

16. Le système selon l'une des revendications 14 à 15, où l'unité de détection/détermination (130) est de plus configurée pour déterminer un type de tête de vibration en comparant le paramètre de stockage d'énergie détecté avec une pluralité de plages de paramètres de stockage d'énergie.

17. Le système selon la revendication 16, comprenant de plus :
une unité de stockage (150) pour stocker une pluralité de paramètres de configuration dépendant d'un type de tête de vibration ;
où l'unité de contrôle (120) est de plus configurée pour sélectionner, basé sur le type de tête de vibration déterminé, l'un des paramètres de configuration dépendant du type de tête de vibration stockés, et de contrôler la tête de vibration (210) du générateur d'aérosol (200) pour générer un aérosol basé sur le paramètre de configuration dépendant du type de tête de vibration sélectionné.

18. Un générateur d'aérosol (200), comprenant :
un boîtier, et
une tête de vibration (210) selon l'une des revendications 1 à 6 ; où la tête de vibration (210) est au moins partiellement logée dans le boîtier et le boîtier inclut de plus un réservoir de fluide.
